# EUROPEAN PATENT SPECIFICATION

(11) **EP 1 250 350 B1**
(45) Date of publication and mention of the grant of the patent: **19.04.2006**
(21) Application number: 01942633.7
(22) Date of filing: 22.01.2001
(51) Int. Cl.: C07J 9/00, C11B 13/00, A61K 31/575, A61P 3/06, A61P 9/10

(54) **NOVEL CRYSTALLINE COMPOSITES COMPRISING PHYTOSTEROLS AND PHYTOSTANOLS OR DERIVATIVES THEREOF**
KRYSTALLINE ZUSAMMENSETZUNGEN ENTHALTEND PHYTOSTEROLE UND PHYTOSTANOLE ODER DERIVATE DAVON
NOUVEAUX COMPOSITES CRISTALLINS COMPRENANT DES PHYTOSTEROLS ET DES PHYTOSTANOLS OU DES DERIVES DESDITS COMPOSES

(30) Priority: 21.01.2000 US 489541
(43) Date of publication of application: 23.10.2002
(60) Divisional application: 05027573.4
(73) Proprietor: Forbes Medi-Tech Inc., Vancouver, British Columbia V6C 2T8 (CA)
(72) Inventor: STEWART, David, John, North Vancouver, British Columbia V7G 2P (CA)
(74) Representative: Jones, Helen M.M.
(86) International application number: PCT/CA2001/000053
(87) International publication number: WO 2001/053320

(56) References cited:
- EP-A- 0 990 662
- WO-A-00/64921
- WO-A-00/64922
- WO-A-00/64924
- WO-A-98/06405
- WO-A-98/19556
- WO-A-99/48907
- SUGANO M ET AL: "COMPARISON OF HYPOCHOLESTEROLEMIC ACTIVITY OF SITOSTEROL AND BETA-SITOSTANOL IN RATS" JOURNAL OF NUTRITION,US,WISTAR INSTITUTE OF ANATOMY AND BIOLOGY, PHILADELPHIA, PA,, vol. 107, 1977, pages 2011-2019, XP000864571 ISSN: 0022-3166
- DATABASE CHEMABS [Online] CHEMICAL ABSTRACTS SERVICE, COLUMBUS, OHIO, US; JIN, QI ET AL: "Extracting phytosterol from sulfate soap using ethyl acetate" retrieved from STN Database accession no. 121:182393 XP002170200 & LINCHAN HUAXUE YU GONGYE (1993), 13(1), 51-6 ,
- SUGANO M ET AL: "LIPID LOWERING ACTIVITY OF PHYTOSTANOLS IN RATS" ATHEROSCLEROSIS, vol. 24, no. 1-2, 1976, pages 301-309, XP001011252 ISSN: 0021-9150
- LING W H ET AL: "ENHANCED EFFICACY OF SITOSTANOL-CONTAINING VERSUS SITOSTANOL-FREE PHYTOSTEROL MIXTURES IN ALTERING LIPOPROTEIN CHOLESTEROL LEVELS ANDSYNTHESIS IN RATS" ATHEROSCLEROSIS,NL,AMSTERDAM, vol. 118, no. 2, 1995, pages 319-331, XP002044615 ISSN: 0021-9150

## Description

### FIELD OF THE INVENTION

This present invention relates to the field of phytosterols and phytostanols and their use in treating and preventing cardiovascular disease, its underlying conditions such as atherosclerosis, hypercholesterolemia, and hyperlipidemia and other disorders.

### BACKGROUND OF THE INVENTION

While recent advances in science and technology are helping to improve quality and add years to human life, the prevention of atherosclerosis, the underlying cause of cardiovascular disease ("CVD") has not been sufficiently addressed. Atherosclerosis is a degenerative process resulting from an interplay of inherited (genetic) factors and environmental factors such as diet and lifestyle. Research to date suggest that cholesterol may play a role in atherosclerosis by forming atherosclerotic plaques in blood vessels, ultimately cutting off blood supply to the heart muscle or alternatively to the brain or limbs, depending on the location of the plaque in the arterial tree (1,2). Overviews have indicated that a 1% reduction in a person's total serum cholesterol yields a 2% reduction in risk of a coronary artery event (3). Statistically, a 10% decrease in average serum cholesterol (e.g. from 6.0 mmol/L to 5.3 mmol/L) may result in the prevention of 100,000 deaths in the United States annually (4).

Sterols are naturally occurring compounds that perform many critical cellular functions. Phytosterols such as campesterol, stigmasterol and beta-sitosterol in plants, ergosterol in fungi and cholesterol in animals are each primary components of cellular and sub-cellular membranes in their respective cell types. The dietary source of phytosterols in humans comes from plant materials i.e. vegetables and plant oils. The estimated daily phytosterol content in the conventional western-type diet is approximately 60-80 milligrams in contrast to a vegetarian diet which would provide about 500 milligrams per day.

Phytosterols have received a great deal of attention due to their ability to decrease serum cholesterol levels when fed to a number of mammalian species, including humans. While the precise mechanism of action remains largely unknown, the relationship between cholesterol and phytosterols is apparently due in part to the similarities between the respective chemical structures (the differences occurring in the side chains of the molecules). It is assumed that phytosterols displace cholesterol from the micellar phase and thereby reduce its absorption or possibly compete with receptor and/or carrier sites in the cholesterol absorption process.

Over forty years ago, Eli Lilly marketed a sterol preparation from tall oil and later from soybean oil called Cytellin™ which was found to lower serum cholesterol by about 9% according to one report (5). Various subsequent researchers have explored the effects of sitosterol preparations on plasma lipid and lipoprotein concentrations (6) and the effects of sitosterol and campesterol from soybean and tall oil sources on serum cholesterols (7). A composition of phytosterols which has been found to be highly effective in lowering serum cholesterol is disclosed in US Patent Serial No. 5,770,749 to Kutney et al. and comprises no more than 70% by weight beta-sitosterol, at least 10% by weight campesterol and stigmastanol (beta-sitostanol). It is noted in this patent that there is some form of synergy between the constituent phytosterols, affording even better cholesterol-lowering results than had been previously achieved.

Despite the obvious and now well recorded advantages of phytosterols, not only in the treatment of CVD and its underlying conditions such as hypercholesterolemia, hyperlipidemia, atherosclerosis, hypertension, thrombosis but in the treatment of other diseases such as Type II diabetes, dementia cancer and aging, the administration of phytosterols and the incorporation thereof into foods, pharmaceuticals and other delivery vehicles has been complicated by the fact that they are highly hydrophobic (i.e.they have poor water solubility) and they are generally also poorly soluble in oils. The provision of a water and oil-soluble phytosterol derivative which could be administered orally and which could be incorporated without further modification into delivery vehicles would be highly desirable.

It is an object of the present invention to obviate or mitigate the above disadvantages.

### SUMMARY OF THE INVENTION

The present invention provides a composite crystalline structure comprising a phytosterol and a phytostanol, or a derivative of either.

The present invention further comprises a composition for treating or preventing CVD and its underlying conditions including atherosclerosis, hypercholesterolemia, hyperlipidemia, hypertension, thrombosis, and related diseases such as Type II diabetes, as well as other diseases that include oxidative damage as part of the underlying disease process such as dementia, aging, and cancer which comprises one or more composite crystalline structures comprising phytosterols or phytostanols, or derivatives thereof and a pharmaceutically acceptable or food quality carrier or adjuvant therefor.

The present invention further provides foods, beverages and nutraceuticals supplemented with one or more of the composite crystalline structures described herein.

The present invention further provides a method for the manufacture of a product for use in treating or preventing CVD and its underlying conditions including atherosclerosis, hypercholesterolemia, hyperlipidemia, hypertension, thrombosis, and related diseases such as Type II diabetes, as well as other diseases that include oxidative damage as part of the underlying disease process such as dementia, ageing, and cancer by administering to an animal one or more of the composite crystalline structures as described herein.

The present invention further provides a process of preparing a composite crystalline structure comprising a phytosterol and a phytostanol, or an esterified derivative of either, which comprises:
a) dissolving the phytosterol purified from its source and the phytostanol purified from its source in a solvent at an ambient temperature or temperature above ambient but lower than the boiling point of the solvent;
b) cooling the solvent to allow crystal formation; and
c) filtering and washing the crystals so formed.

What is achieved within the scope of the present invention is the creation of a composite crystalline structure wherein a phytosterol and a phytostanol, under appropriate conditions, are recrystallized together forming a unitary structure which is a single composite endotherm as evidenced using differential scanning calorimetry ("DSC"). In addition, these new crystalline structures have their own x-ray powder diffraction ("XRD") profiles, their own chemical characteristics and their own particular melting points as compared to each of the individual phytosterols or phytostanol alone or together in a composition. In other words, what is created is a structure chemically and physically unlike either of the phytosterol or phytostanol starting materials alone, or mixed in composition.

These crystalline composites have numerous advantages over individual phytosterols or phytostanols or mixtures thereof previously known and described in the art. In particular, it has been found that solubility in oils, oil-based media and fats is improved by a method which is simple, economical and which does not require extensive prior modifications to the sterols or stanols, such as by esterification. Naturally occurring and isolated phytosterols and stanols are in coarse, powder, crystalline form which is not amenable to the formation of a homogeneous mixture in water, oils or fats, without some type of modification. Although esterification of phytosterols and stanols does make them considerably more soluble in fats and oils and is a widely used technique for practical reasons, these esterified derivatives do not inhibit the absorption of cholesterol as effectively as free sterols (8). In a number of prior patents, stanols, in particular, are esterified in order to enhance their poor solubility (see US Patent Serial No. 5,502,045 to Raision Tehtaat Oy AB). The disadvantages of phytosterol/stanol fatty acid esters include the following: decreased absorption of lipophilic micronutrients (like beta-carotene) (9) and increased processing time and costs to prepare. Heating beta-sitosterol in oil is described in PCT/FI99/00121; however, the resultant product is one in which the sterols are only partially dissolved.

Within the scope of the present invention, naturally occurring phytosterols and phytostanols in crystalline form, are re-crystallized together to form unitary composite crystalline structures which not only exhibit enhanced solubility in oils and fats as compared to individual phytosterols or phytostanols or mixtures thereof previously known and described in the art, but also show greater efficacy in lowering serum cholesterol.

Accordingly, oil and fat-based compositions comprising the crystalline structures of the present invention can be prepared and used as such or they can be easily incorporated into foods, beverages, dietary supplements, pharmaceuticals and nutraceuticals. This enhanced solubility generally translates into lower concentrations of phytosterols and/or phytostanols that need be provided in the oil or fat in order to achieve the desired therapeutic or dietary effect.

These and other advantages of the present invention are outlined in more detail hereinbelow.

### BRIEF DESCRIPTION OF THE DRAWINGS

The present invention is illustrated by the following non-limiting drawings in which:
Figure 1 is a microscopic photograph of the crystalline material from FCP-3P1;
Figure 2 is another microscopic photograph of the crystalline material from FCP-3P1;
Figure 3 is a microscopic photograph of the crystalline structure from FCP-3P2;
Figure 4 is another microscopic photograph of the crystalline structure from FCP-3P2;
Figure 5 is a microscopic photograph of an equal mixture of FCP-3P1 and FCP-3P2 recrystallized from ethyl acetate;
Figure 6 is another microscopic photograph of an equal mixture of FCP-3P1 and FCP-3P2 recrystallized from ethyl acetate;
Figure 7 is another microscopic photograph of an equal mixture of FCP-3P1 and FCP-3P2 recrystallized from ethyl acetate;
Figure 8 is another microscopic photograph of an equal mixture of FCP-3P1 and FCP-3P2 recrystallized from ethyl acetate;
Figures 9 is the DSC indium calibration data;
Figure 10 is the DSC data overlay for FCP-3P1, FCP-3P2, the equal mixture of FCP 3P1 and FCP-3P2 in composition and the recrystallized material (FM-PH-38);
Figure 11 is the DSC data for FCP-3P2;
Figure 12 is the DSC data for FCP-3P1;
Figure 13 is the DSC data for an equal mixture of FCP-3P1 and FCP-3P2 in composition;
Figure 14 is DSC data for the equal mixture of FCP-3P1 and FCP-3P2 recrystallized from ethyl aectate;
Figure 15 is the X-ray diffraction data for FCP-3P2;
Figure 16 is the X-ray diffraction data for FCP-3P1; and
Figure 17 is the X-ray diffraction data for the equal mixture of FCP-3P1 and FCP-3P2 recrystallized from ethyl acetate.

### PREFERRED EMBODIMENTS OF THE INVENTION

According to one embodiment the present invention, there is provided a composite crystalline structure comprising a phytosterol and a phytostanol, or a derivative of either suitable for use per se in treating or preventing CVD and its underlying conditions, such as atherosclerosis, hypercholesterolemia, hyperlipidemia, hypertension, thrombosis, and related diseases such as Type II diabetes, as well as in treating and preventing other diseases that include oxidative damage as part of the underlying disease process such as dementia, aging, and cancer.

At the outset, it is to be understood that throughout this specification, the terms "composite crystalline structure", "crystalline composite structure", "new crystalline structures", "composite crystals", "novel structures", "composite" and "crystal structures" are used interchangeably and are intended, unless otherwise clearly stated, to possess the same meaning.

### Phytosterols/Phytostanols

The term "phytosterol", within the scope of the present invention, includes all phytosterols without limitation, for example: sitosterol, campesterol, stigmasterol, brassicasterol, desmosterol, chalinosterol, poriferasterol, clionasterol and all natural or synthesized forms and derivatives thereof, including isomers. The term "phytostanol" includes all saturated or hydrogenated phytosterols and all natural or synthesized forms and esterified derivatives thereof, including isomers. It is also to be understood that, when in doubt throughout the specification, the term "phytosterol" encompasses both phytosterol and phytostanol i.e. the terms may be used interchangeably unless otherwise specified.

The phytosterols and phytostanols for use in forming the crystalline composite structures in accordance with this invention may be procured from a variety of natural sources. For example, they may be obtained from the processing of plant oils (including aquatic plants) such as corn oil and other vegetable oils, wheat germ oil, soy extract, rice extract, rice bran, rapeseed oil, sesame oil and fish oils. Without limiting the generality of the foregoing, it is to be understood that there are other sources of phytosterols and phytostanols such as marine animals from which the composition of the present invention may be prepared. US Patent Serial No. 4,420,427 teaches the preparation of sterols from vegetable oil sludge using solvents such as methanol. Alternatively, phytosterols and phytostanols may be obtained from tall oil pitch or soap, by-products of forestry practises as described in US Patent Serial No.5,770,749. Phytosterols and phytostanols are widely available through commercial supply companies.

Without limiting the generality of the foregoing, it is to be understood that there are numerous known methods of extracting and purifying phytosterols and phytostanols from the "source" of choice and the present invention is not limited to any one method of attaining these purified phytosterols and phytostanols. Generally, however, the phytosterols and phytostanols, purified from the source, are in a crystalline form, with each sterol and stanol represented by an individual crystal structure, and having its own DSC endotherm and XRD profile.

In a most preferred form, the crystalline composite structure of the present invention is formed with naturally-derived or synthesized beta-sitosterol or campesterol and either sitostanol or campestanol, or mixtures thereof.

### Recrystallization Process

In a preferred form, the recrystallization process is as follows:
a) one or more phytosterols and phytostanols are selected for combination into the unique crystalline structure;
b) these phytosterols and phytostanol each purified from its source are dissolved in a solvent at an ambient temperature or temperature above ambient but lower than the boiling point of the solvent;
c) the solvent is cooled to allow crystal formation; and
d) the crystals so formed are filtered and dried.

Many solvents are suitable for use within the method of the present invention. Most preferably, the solvent is selected from any one of the following, alone or in combination:
1) any hydrocarbon solvent such as pentane, hexane and higher family members from C7 to C10 inclusive, or derivatives thereof;
2) any alcohol of the family C1 to C10 inclusive, including without limitation, ethanol, isopropyl alcohol and methanol, or derivatives thereof;
3) aromatic solvents such as toluene and xylene and derivatives thereof;
4) ketones having from 1 to 10 carbon atoms, including, without limitation, acetone, propanone, methylisobutyl ketone and butanone, or derivatives thereof; and
5) esters such as ethyl acetate and those derived from carboxylic acids of the family C2 to C10 inclusive.

In one preferred form, the phytosterols and phytostanols are dissolved in the solvent at ambient room temperature from 18 to 30 ° C, although it is not intended that the process be limited to any particular temperature. For example, when isopropyl alcohol is used as the recrystallization solvent, the temperature for dissolution of the phytosterols and phytostanols is most preferably in the range of 50-80° C. What is critical is that substantially all of the phytosterols and phytostanols are dissolved in the chosen solvent (the solvent is "saturated") and that the temperature chosen is lower than the boiling point of the selected solvent. This dissolution may take anywhere from 30 minutes to 6 hours depending on the solvent used and the constituent phytosterols/stanols. Likewise, the cooling temperature of step b), to promote formation of the crystals may vary considerably but it is generally from 1 to 20° C. Preferably, the cooling occurs rapidly in less than one hour and most preferably in about 30 minutes to a temperature at which crystal formation begins. The resulting slurry is best stirred slowly during this process. After the sterols/stanols have precipitated from the solution, the crystals so formed are filtered and repeatedly washed (for example with deionized water), and then lightly compressed and suctioned free of liquid.

Once recrystallization is complete, to confirm the formation of new crystalline structures, endotherm analysis using DSC technology and XRD profiles or other techniques in which the molecular structure or crystalline matrix can be checked. What has been found is that the new crystal structures so formed have properties (exhibited as XRD and DSC data) which are unlike each of the starting materials alone (the particular phytosterols or phytostanols) or in composition and which are, in fact, intermediate between the starting materials' properties.
In one embodiment, the composite crystal of the present invention may be used directly and without any further adaptations or work-ups in the therapeutic and dietary protocols outlined below. In another embodiment, the composite crystal may be formed into a solid powder through precipitation, filtration and drying, spray drying, lyophilization or by other conventional work-up techniques. This powder form may then be used in the therapeutic and dietary protocols noted below.

The method of the present invention, in which phytosterols and phytostanols are recrystallized together to form new crystalline structures may be used:
1) to form new "designer" compositions of phytosterols and phytostanols having any desired concentration range of the constituents; or
2) to blend or "spike" phytosterols and phytostanols into sterol compositions extracted from natural sources in order to ensure specification uniformity between batches.

"Designer" compositions can be prepared, under controlled conditions, having any concentration of phytosterols and phytostanols desired. For example, a composition having 99% by weight ("w/w") phytosterols and 1 % w/w phytostanols or anywhere in between or having 99% w/w phytostanols and 1 % w/w phytosterols or anywhere in between can be prepared using the recystallization protocol. Accordingly, there is afforded a great deal of processing flexibility as the relative amounts of each sterol and stanol in the final composition can be adjusted by successive recrystallizations. For example, if a higher relative concentration of sitostanol is desired in a given composition, sitostanol may be recrystallized with the sterol/stanols already in the composition yielding one or more new composite crystal structures within that composition. Preferably, the designer composition of the present invention, prepared by recrystallization comprises 20 to 80% w/w phytosterols and from 80 to 20% w/w phytostanols, more preferably from 30 to 70% w/w phytosterols and from 70 to 30 w/w phytostanols, more preferably from 40 to 60% w/w phytosterols and from 60 to 40% w/w phytostanols and most preferably 50% w/w phytosterols and phytostanols.

Blending or spiking phytosterols and phytostanols into compositions extracted from natural sources also has a number of critical commercial applications. Regardless of the natural source from which the phytosterols and phytostanols are extracted and purified, there will always be some variation, batch to batch, in the relative amounts of each phytosterol and phytostanol. This can be a production problem when it is necessary consistently to produce a composition having a particular set of specifications. The recrystallization method of the present invention addresses and solves this problem. For example, if one extracted and purified batch does not comply with the necessary specification in that the concentration of sitostanol is too low, this can readily be adjusted by recrystallizing into the composition a set amount of sitostanol to "spike" up the level.

Both the formation of designer compositions and the spiking of existing natural compositions can be achieved with phytosterols and phytostanols originally derived from any source (without limitation, tall oil soap, tall oil pitch, vegetable distillate, soybean extract and the like). In addition, it is fully contemplated that phytosterol and phytostanol sources can be "mixed". In other words, a composition naturally derived from tall oil soap may be "spiked" and recrystallized with a sterol or stanol derived from soybean extract. The "spiking" of compositions is described in more detail in the Examples.

### Advantages of Novel Crystal Composite Structures

The novel structures of the present invention afford many dietary and therapeutic advantages when compared to the use of phytosterols/phytostanols alone or together in composition. What is primarily achieved by the formation of the composite crystals is enhanced solubility of the phytosterol/stanol constituents in oils and fats, without the need for chemical or other structural modifications to the sterols and stanols. Not only does this facilitate the incorporation of sterols and stanols into "delivery" vehicles (with the attendant cost and time savings) but this new unitary structure is more readily absorbed in bile acid micelli, thereby efficiently displacing cholesterol and blocking its absorption. Both phytosterols and phytostanols must first be dissolved in oil micelles in the intestine before interaction with cholesterol can occur.

For reasons that are as yet unclear, the crystal structures of the present invention, in which phytosterols and phytostanols are formed into unitary structures, maximize many of the individual advantages of phytosterols and phytostanols, while minimizing the respective disadvantages. For example, many studies suggest that stanols in general, and sitostanol in particular, are the most effective inhibitors of cholesterol absorption, however, they have poor lipid solubility (10). Phytosterols are generally more soluble but are less effective at cholesterol lowering (11, 12 and 13). The crystal structures of the present invention are more soluble in oils and fats than phytosterols i.e. they exhibit an oil solubility in between that of phytosterols and phytostanols and surprisingly, they exhibit cholesterol lowering efficacy more comparable to stanols than sterols. This combination allows a more effective action in lowering cholesterol than either phytosterols or phytostanol alone or in composition together.

### Methods of Use

The structures of the present invention may be used directly and without further modification in cooking, baking and the like as agents to lower serum cholesterol in animals, particularly humans. They may be added to any edible oil and used for cooking, baking, and general use. Alternatively, the structures may be treated to enhance delivery into various other delivery media. For example, the crystal structures may be physically modified as described in International Application No. PCT/CA99/00402, specifically pages 11-23, (published on November 25, 1999 and incorporated herein by reference) to enhance even further the solubility and dispersability of the phytosterol and/or phytostanol in the chosen delivery medium.

In addition, the present invention fully contemplates the formation of oleaginous gel foodstuffs such as peanut butter, mayonnaise, ice cream and margarine spreads incorporating such structures. Further, the composite crystals can readily be included in a variety of low fat foods such as yoghurts in which the fine crystals disperse evenly in the product. There are numerous modes or "vehicles" of delivery of this composition, accordingly, this invention is not intended to be limited to the following delivery examples.

### 1) Pharmaceutical Dosage Forms:

It is contemplated within the scope of the present invention that the structures of the present invention may be incorporated into various conventional pharmaceutical preparations and dosage forms such as tablets (plain and coated) for use orally, bucally or lingually, capsules (hard and soft, gelatin, with or without additional coatings) powders, granules (including effervescent granules), pellets, microparticulates, solutions (such as micellar, syrups, elixirs and drops), lozenges, pastilles, ampuls, emulsions, microemulsions, ointments, creams, suppositories, gels, and transdermal patches, modified release dosage forms together with customary excipients and/or diluents and stabilizers.

The structures of the present invention, adapted into the appropriate dosage form as described above may be administered to animals, including humans, orally, by injection (intra-venously, subcutaneously, intra-peritoneally, intra-dermally or intramuscularly), topically or in other ways. Although the precise mechanism of action is unclear, the structures of the present invention, administered intra-venously, lower serum cholesterol. It is believed that some blends of phytosterols, in concert, may have, in addition to the role as an inhibitor of cholesterol absorption in the intestine, a systemic effect on cholesterol homeostasis through bile acid synthesis, enterocycte and biliary cholesterol excretion, bile acid excretion and changes in enzyme kinetics and cholesterol transport between various compartments within the body (PCT/CA97/00474 which was published on January 15, 1998).

The structures as described herein may be used in both dietary and therapeutic capacities in order to treat and/or prevent CVD, its underlying conditions such as hypercholesterolemia, hyperlipidemia, arteriosclerosis, hypertension, thrombosis, related diseases such as Type II diabetes, as well as other diseases that include oxidative damage as part of the underlying disease process such as dementia, aging, and cancer. In populations, which are considered "high-risk" for CVD or any of the oxidation related disorders, it is contemplated that the compositions and foodstuffs in which they are contained be used in primary, secondary and tertiary treatment programs.

In order to appreciate the various possible vehicles of the delivery of the compositions, the list below is provided. The doses of the derivatives will vary depending upon, among other factors, the mode of delivery (i.e. how and into which food or beverage or pharmaceutical the derivatives are ultimately incorporated), the patient size and condition, the result to be achieved, as well as other factors known to those skilled in the art of food additives and medicinal agents. Generally, however, it is preferred that the derivatives of the present invention be administered to humans in a form comprising up to 6 grams (based on a 70kg person) of phytosterols and/or phytostanols per day, more preferably from 1-5 grams per day and most preferably 1.5 grams per day. It will also be recognized that the provision of much larger daily doses of the derivatives are not harmful to the animal host, as excess will simply pass through normal excretory channels.

### 2) Foods/Beverages/Nutraceuticals:

In another form of the present invention, the structures of the present invention may be incorporated into foods, beverages and nutraceuticals, including, without limitation, the following:
1) Dairy Products --such as cheeses, butter, milk and other dairy beverages, spreads and dairy mixes, ice cream and yoghurt;
2) Fat-Based Products--such as margarines, spreads, mayonnaise, shortenings, cooking and frying oils and dressings;
3) Cereal-Based Products--comprising grains (for example, bread and pastas) whether these goods are cooked, baked or otherwise processed;
4) Confectioneries-such as chocolate, candies, chewing gum, desserts, non-dairy toppings (for example Cool Whip™), sorbets, icings and other fillings;
5) Beverages-- whether alcoholic or non-alcoholic and including colas and other soft drinks, juice drinks, dietary supplement and meal replacement drinks such as those sold under the trade-marks Boost™ and Ensure™; and
6) Miscellaneous Products--including eggs and egg products, processed foods such as soups, pre-prepared pastas

The optimal ratio of phytosterols to phytostanol in the composite depends, to some extent, on the matrix in which the composite is to be suspended or incorporated. Generally, foods with a high fat content will accommodate a higher proportion of phytostanols than lower fat foods. This can be determined empirically with each type of food/beverage in which it is desired to add the composite.

The structures of the present invention may be incorporated directly and without further modification into the food, nutraceutical or beverage by techniques such as mixing, infusion, injection, blending, dispersing, emulsifying, immersion, spraying and kneading. Alternatively, these structures may be applied directly onto a food or into a beverage by the consumer prior to ingestion. These are simple and economical modes of delivery.

While the following examples are intended to illustrate various aspects of the present invention and to assist in the preparation of the structures, they are not intended to limit the scope of invention as claimed herein.

### EXAMPLE 1

### Recrystallization from Ethyl Actetate

An equal mixture (1:1 by weight) of a non-hydrogenated tall oil derived composition comprising primarily campesterol and beta-sitosterol (hereinafter called FCP-3P1) and a hydrogenated tall oil derived phytosterol composition comprising primarily sitostanol and campestanol (hereinafter called FCP-3P2) was dissolved in ethyl acetate at ambient room temperaure (21° C) to achieve saturation solution. Phytosterol was recrystallized from the saturated ethyl acetate solution at approximately 6° C overnight before being filtered onto No.1 Whatman filter paper using a Buchner glass filter funnel with vacuum suction. The recrystallized material was dried at 35° C under open atmosphere in a hot oven for 48 hours before storage in a 50 ml polypropylene screw capped tube. This ethyl acetate recrystallized material from an equal mixture of FCP-3P1 and FCP-3P2 is referred to in some of the Figures as FM-PH-38. An analysis of this new recrystallized product was undertaken using microscopic photography, Differential Scanning Calorimetry ("DSC") and X-ray Diffraction ("XRD") and appropriate comparisons were made between the data for FM-PH-38 and:
1) each of the starting materials alone: FCP-3P1 and FCP-3P2; and
2) and equal mixture of FCP-3P1 and FCP-3P2 in composition i.e. not recrystallized together.

### Microscopic Photography:

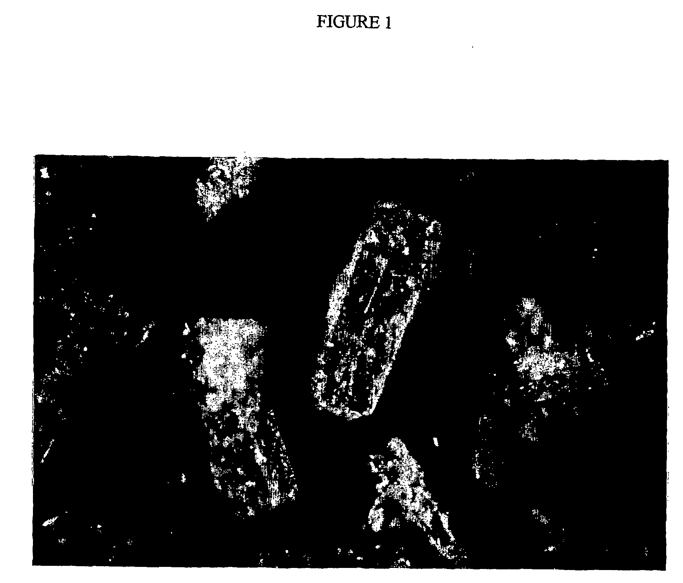

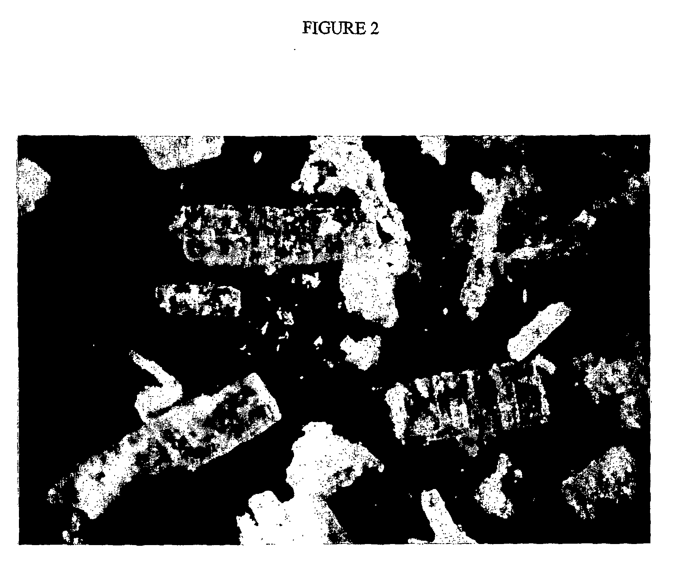

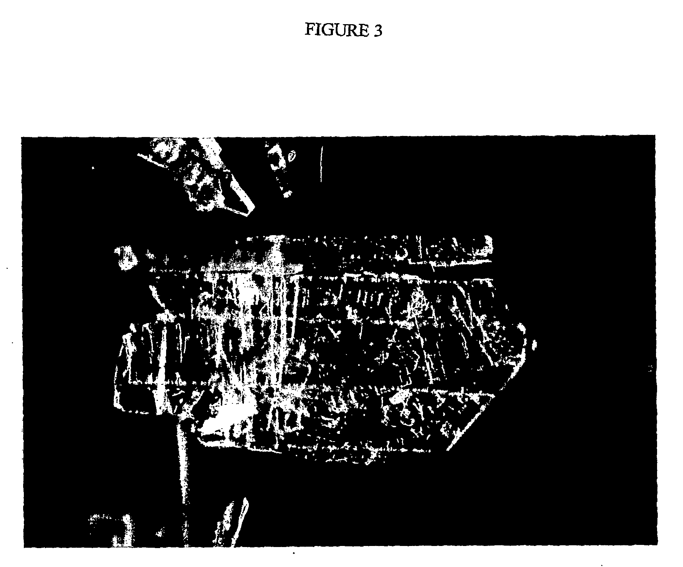

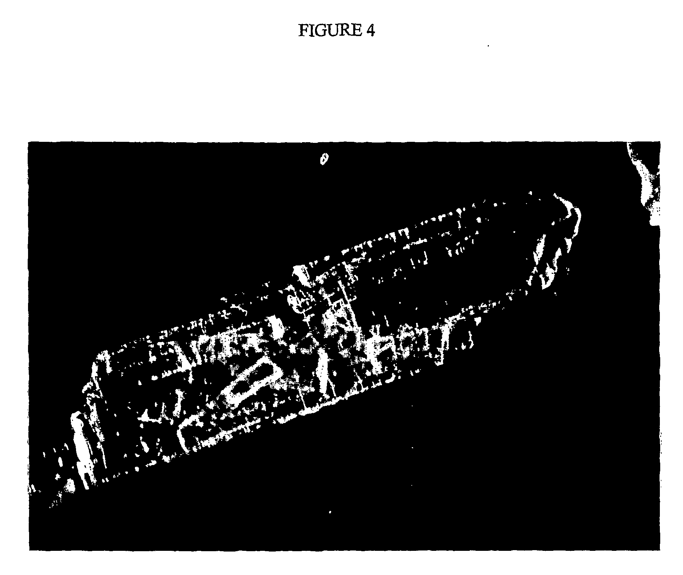

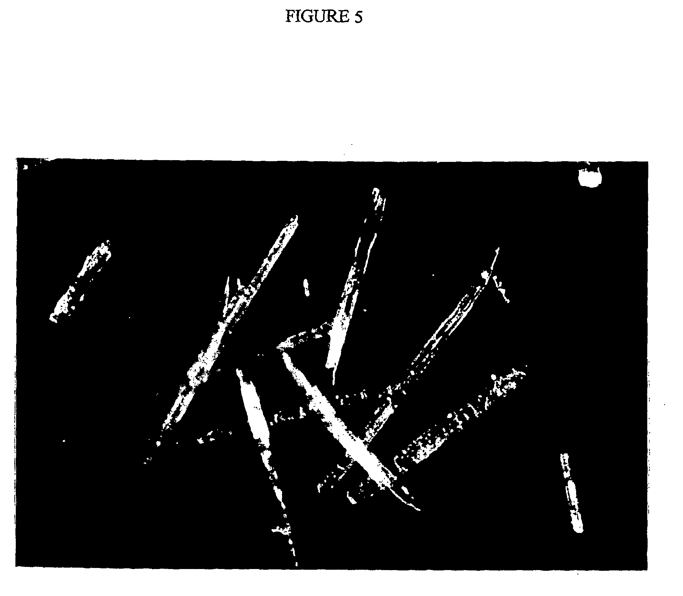

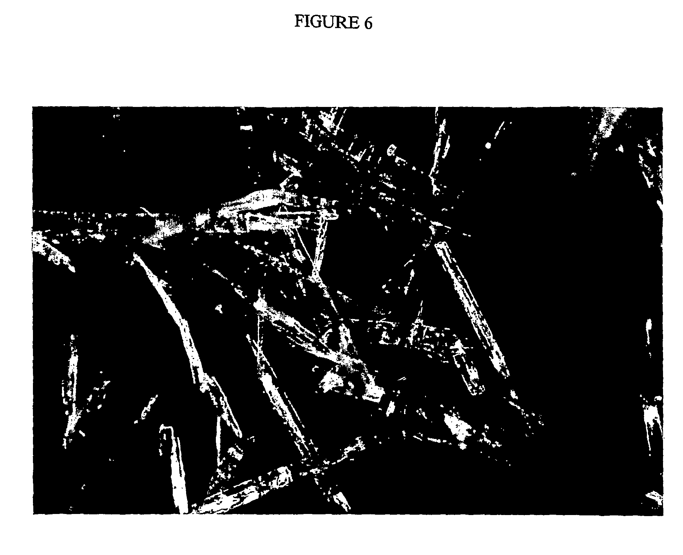

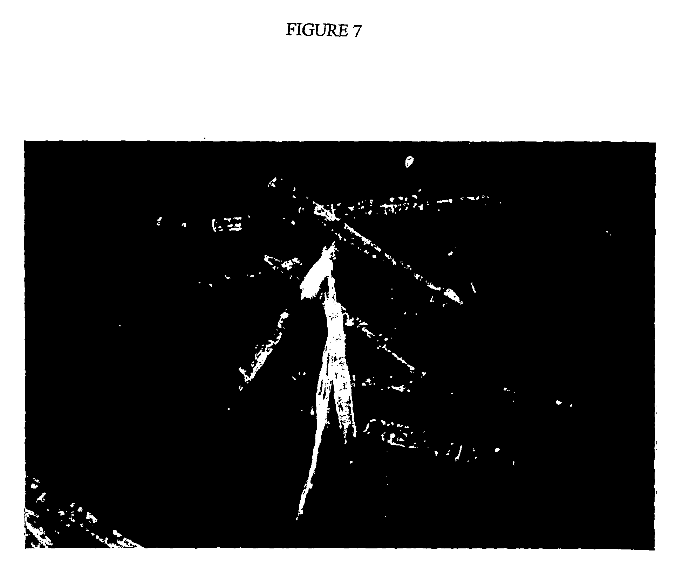

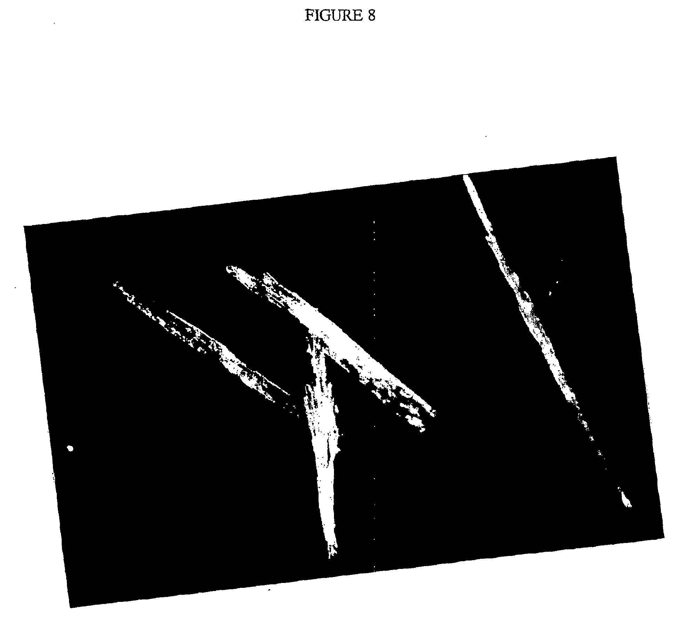

The solid state crystalline structures of FCP-3P1, FCP-3P2 and FM-PH-38 were investigated using microscopic photography at 15X and 40X magnification (although only 40X magnification is shown in the Figures). The individual material was examined under a light microscope and photographic records were obtained against a dark background using black and white film. Records for FCP-3P1 are depicted in Figures 1 and 2. Records for FCP-3P2 are depicted in Figures 3 and 4. Records for recrystallized product FM-PH-38 are depicted in Figures 5 through 8 inclusive.

### Differential Scanning Calorimetry

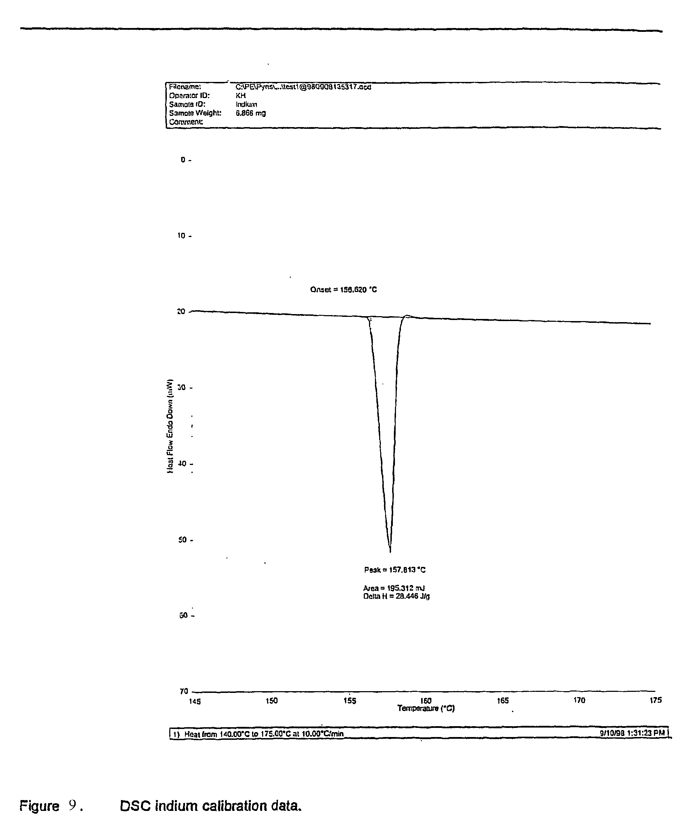

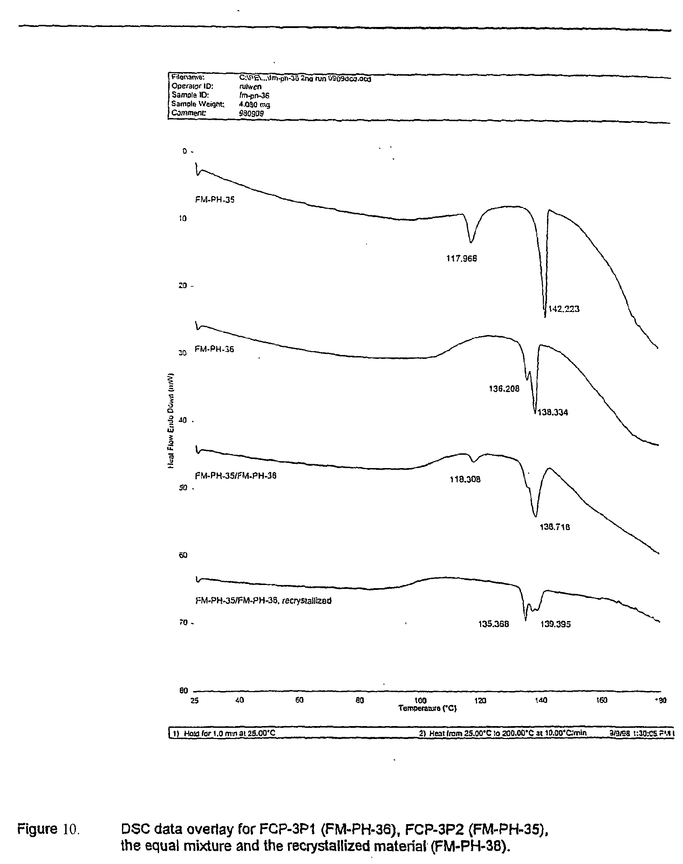

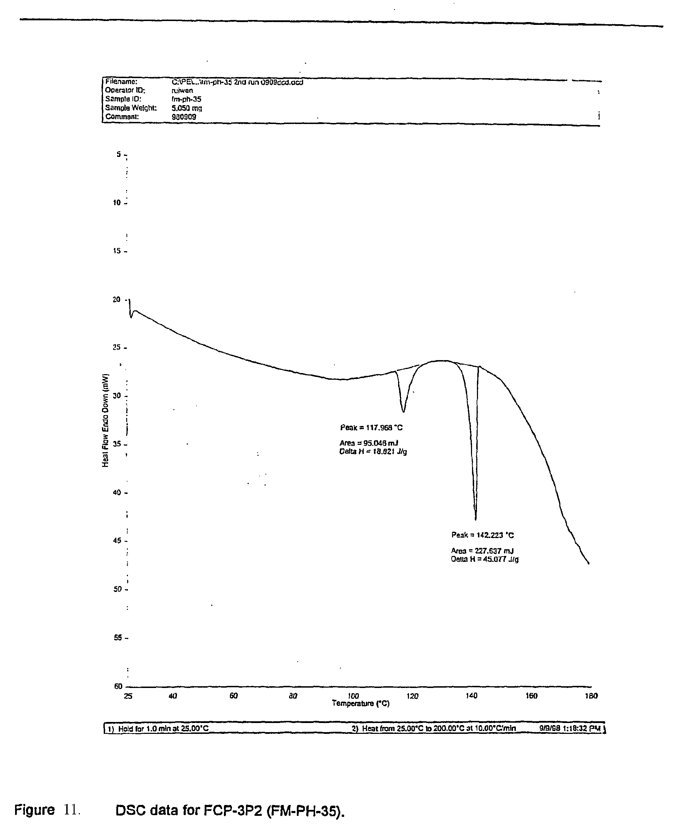

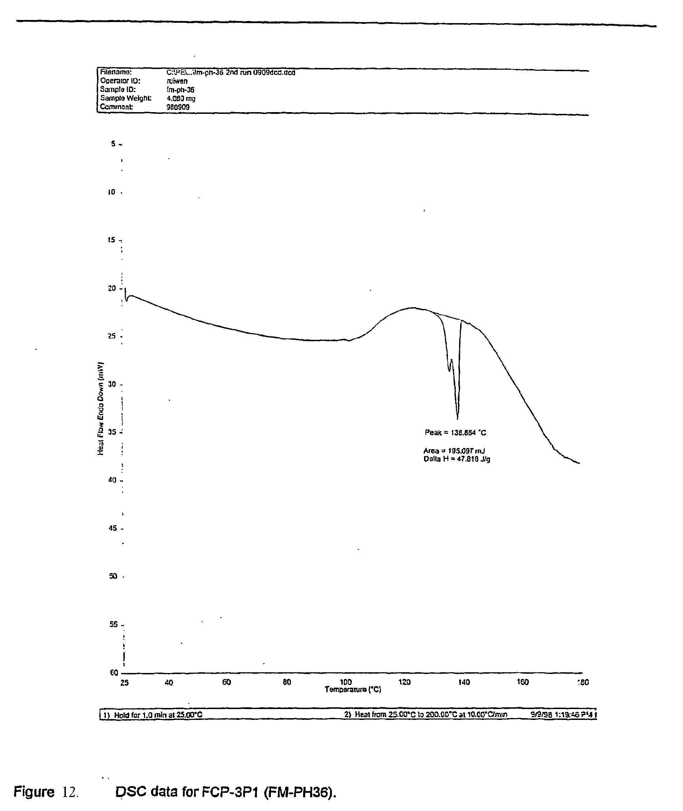

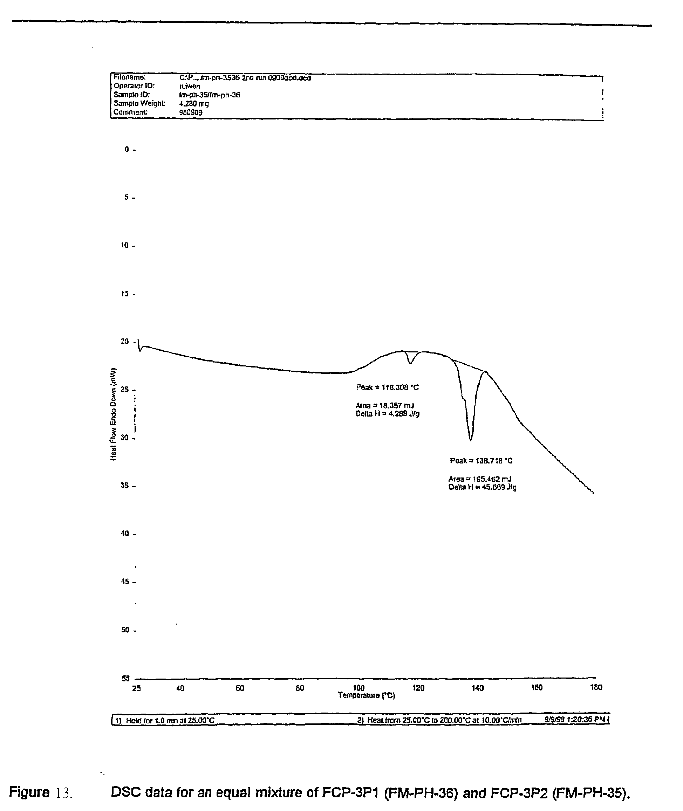

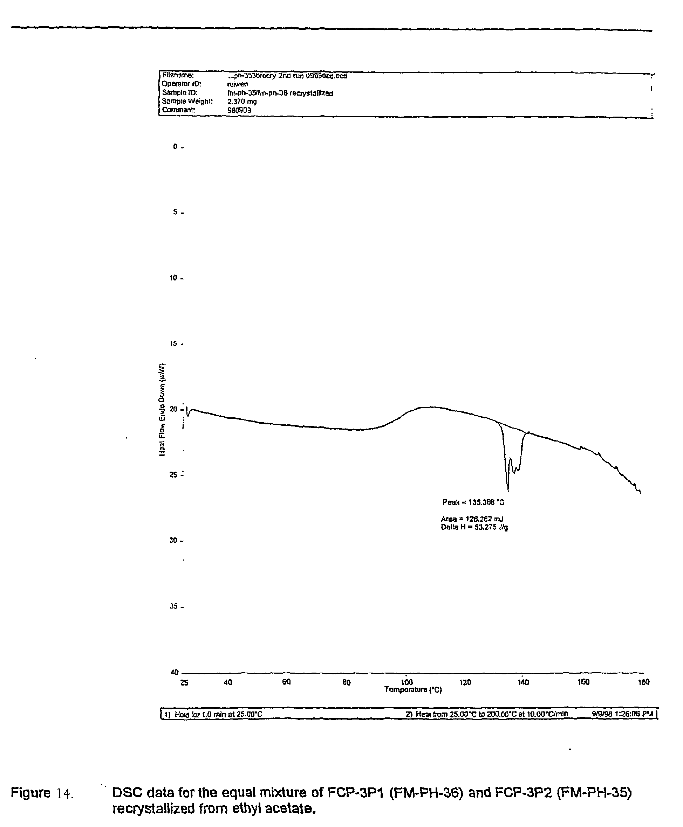

DSC determinations were carried out using a Dupont Model 910S DSC (New Castle, DL) calibrated with indium. Samples weighing between 3 to 4 mg were placed in open aluminum pans (n=3 for each of the test materials). The DSC cell was purged with nitrogen flowing at 40 ml/min. All measurements were made with a scan rate of 10° C/min. Scans were obtained by heating from ambient temperature to 350° C. DSC data for FCP-3P1, FCP-3P2 and FM-PH-38 are presented in Figures 9 through 14.

### X-Ray Diffraction

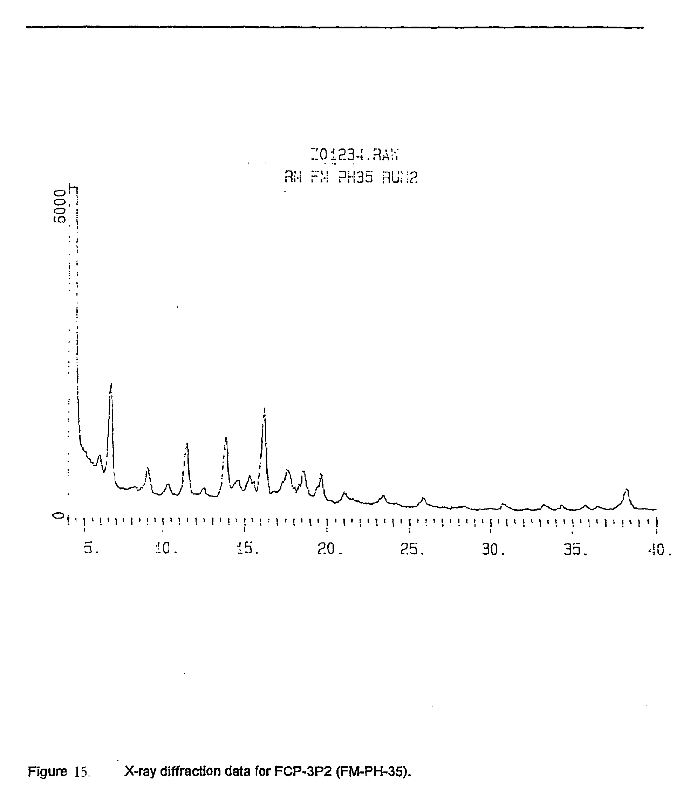

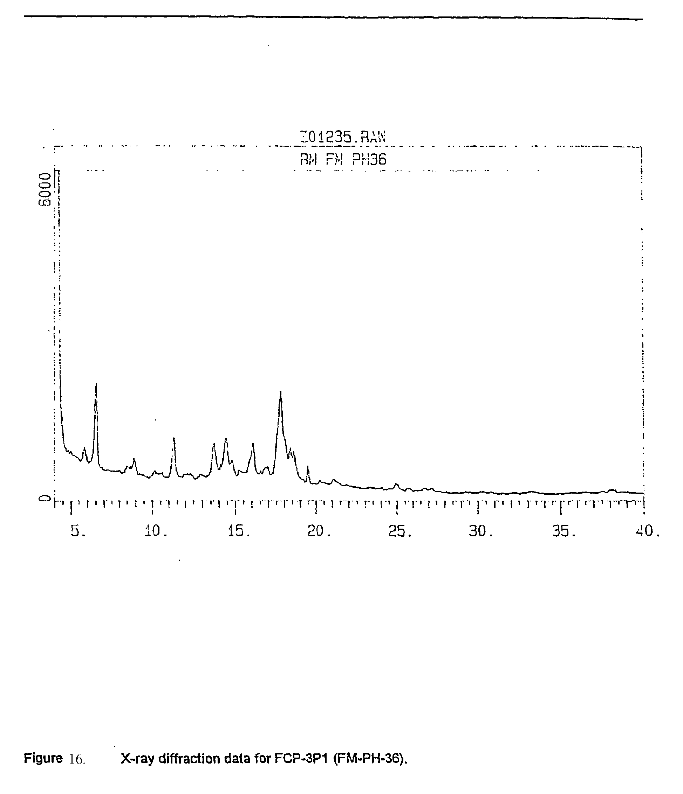

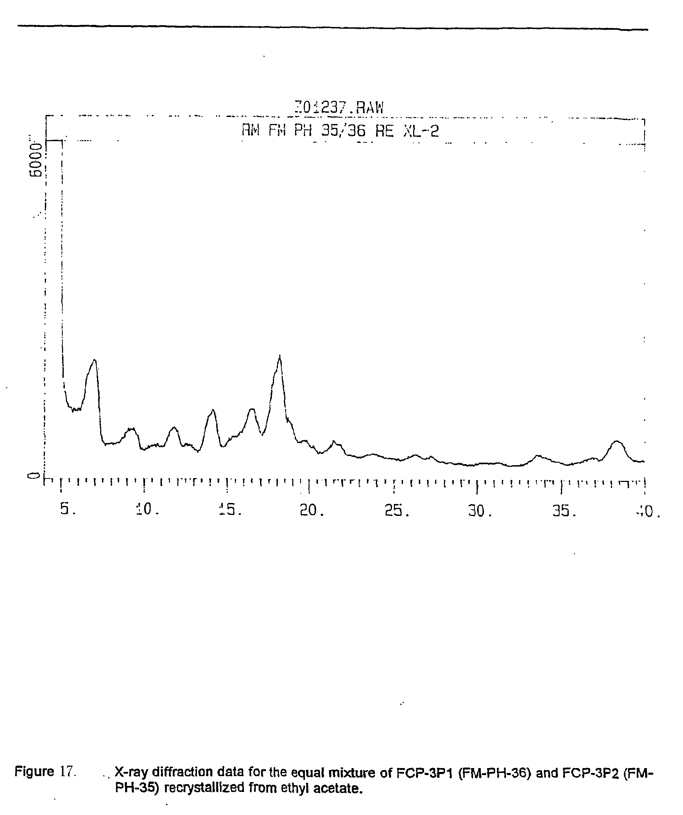

XRD measurements of FCP-3P1, FCP-3P2 and FM-PH-38 were achieved using a wide angle x-ray diffractor and are presented in Figures 15 through 17. Generally, XRD is the classical method to investigate how crystals form and are assembled together.

### Chemical Composition

The features of the chemical composition were investigated using gas chromatography/ mass spectrometry ("GC/MS"). This method involves the use of a bonded phase 5% diphenyl dimethylsiloxane fused silica capillary column for the separation of the four phytosterols. Quantitation of the four major phytosterol components is based on an internal standard ratio approach with the use of cholestane added to each sample as the internal standard. The peak area ratio of each phytosterol to cholestane was plotted against spiked calibration standard concentrations. Calibration standard curves were established from weighted (1/Y) linear regression analysis of peak area ratios against a minimum of 6 phytosterol standard concentrations over a range from 0.01 to 3.0 mg/ml. A Hewlett Packard Model 5890 series II gas chromtograph was operated with model 5971 GC/MS (MSD) and a model 3635 autosampler using the following parameters:

| | |
|---|---|
| GC column | 30m x 0.25mm x 0.25 um SAC-5 |
| GC oven temp | 270 degrees C |
| GC column carrier gas | helium |
| GC/MSD transfer line temp | 300 degrees C |
| Injector temp | 300 degrees C |
| Injection volume | 1 ul |
| Injection mode | splitless (purge time 0.5 minutes) |

### Results and Discussion:

### Photomicrographs:

Differences in appearance of the crystal outline between FCP-3P1, FCP-3P2 and FM-PH-38 in Figures 1 through 8 show that these three materials are chemically unique. FM-PH-38 crystals in Figures 5 through 8 clearly show thinner rods. Although the clarity seems to be similar to FCP-3P2, the shape is not at all similar to the block-shaped crystals shown in Figures 3 and 4.

### DSC:

The data is presented in its entirety in Figures 9 through 13 but is summarized in Table 1:

**Table 1**

| Batch # | DCS Endotherms |
|---|---|
| Indium calibration standard | 157.81 (calibration correct) |
| FCP-3P2 | 117.97 (broad peak) 142.22 (sharp peak) |
| FCP-3P1 | 136.21, 138.33 (broad duplet) |
| FM-PH-38 | 135.7, 139.39 (broad triplet) |
| Composition of 3P1 and 3P2 | 118.31 (broad peak), 138.72 (broad duplex shoulder) |

Melting points for individual sterols and stanols, in degrees C are as follows:

| | |
|---|---|
| Beta-sitosterol | 140 |
| Sitostanol | 138-139 |
| Campesterol | 157-158 |
| Campestanol | 146.5-147.8 |

The DSC endotherms profiled in Figures 9 through 13 indicated that the four major components interact during melting and do not directly or simply correlate with the melting points of the individual components. A physical mixture (composition) of FCP-3P1 and FCP-3P2 shows two endotherms at 118.31 °C and 138.72° C as compared to the recrystallized material (FM-PH-38) which demonstrates a single composite endotherm with peaks at 135.37° C and 139.39° C comprising three closely overlapping peaks (triplet). This clearly shows that the two starting materials have combined to produce a composite crystalline structure having its own particular melting properties in comparison with the individual starting materials alone, or in composition.

### XRD:

Data is shown in Figures 15 through 17. Spectra for FM-PH-38 shows no well defined peaks which indicates that there is no well defined crystal order as in most crystalline structures, including FCP-3P1 and FCP-3P2. Overall, the crystal habit of FM-PH-38 is different from the other materials.

### Chemical Composition:

The GC/MS results indicating the proportion of the four major phytosterols in the three materials are as follows:

**Table 2-Summary of Four Major Phytosterols**

| Phytosterols | FCP-3P1 | FCP-3P2 | Recrystallized product |
|---|---|---|---|
| Campesterol | 0.17 | 0.01 | 0.09 |
| Campestanol | 0.05 | 0.13 | 0.17 |
| Beta-sitosterol | 0.40 | 0.03 | 0.20 |

| | | | |
|---|---|---|---|
| Sitostanol | 0.26 | 0.52 | 0.66 |
| % w/w | 88.9 | 107.6 | 103.7 |

**Table 3- GC/MS Quantitation of Major Phytosterols in FCP-3P2**

| Test sample: FM-PH-35 | Sample 1 at 0.70 mg/ml | | Sample 2 at 0.60 mg/ml | | |
|---|---|---|---|---|---|
| | Sample 1, analysis 1 | Sample 1, analysis 2 | Sample 2, analysis 1 | Sample 2, analysis 2 | Mean |
| campesterol | 0.02 | 0.01 | 0.01 | 0.01 | 0.01 |
| campestanol | 0.16 | 0.14 | 0.11 | 0.11 | 0.13 |
| β-sitosterol | 0:03 | 0.03 | 0.03 | 0.03 | 0.03 |
| sitostanol | 0.58 | 0.56 | 0.49 | 0.46 | 0.52 |
| total major sterols | 0.79 | 0.75 | 0.65 | 0.62 | 0.70 |
| %w/w major sterols (wet weight basis). | 112.4 | 106.4 | 107.8 | 103.7 | 107.6 |

**Table 4- GC/MS Quantitation of Major Phytosterols in FCP-3P1**

| Test sample: FM-PH-36 | sample concentration at 0.99 mg/ml | | | |
|---|---|---|---|---|
| | analysis 1 | analysis 2 | analysis 3 | Mean |
| campesterol | 0.18 | 0.17 | 0.17 | 0.17 |
| campestanol | 0.05 | 0.05 | 0.05 | 0.05 |
| β-sitosterol | 0.40 | 0.39 | 0.40 | 0.40 |
| sitostanol | 0.27 | 0.26 | 0.27 | 0.26 |
| | | | | |
| major sterol components | 90.0 | 87.5 | 89.2 | 88.9 |
| (%w/w wet weight basis) | | | | |

**Table 5- GC/MS Quantitation of Major Phytosterols in FM-PH-38**

| FM-PH-35&36 recrystalized | Sample concentration at 1.08 mg/ml | | | |
|---|---|---|---|---|
| | analysis 1 | analysis 2 | analysis 3 | Mean |
| campesterol | 0.09 | 0.09 | 0.07 | 0.09 |
| campestanol | 0.19 | 0.19 | 0.14 | 0.17 |
| β-sitosterol | 0.21 | 0.22 | 0.18 | 0.20 |
| sitostanol | 0.62 | 0.69 | 0.67 | 0.66 |
| major sterol components | 102.9 | 110.6 | 97.7 | 103.7 |
| (%w/w wet weight basis) | | | | |

### EXAMPLE 2

### Recrystallization from Isopropyl Alcohol

An equal mixture (1:1 by weight) of FCP-3P1 and FCP-3P2 was dissolved in isopropyl alcohol at 75° C to achieve saturation solution. Phytosterol was recrystallized from the saturated isopropyl alcohol solution at approximately 6° C overnight before being filtered onto No.1 Whatman filter paper using a Buchner glass filter funnel with vacuum suction. The recrystallized material was dried at 35° C under open atmosphere in a hot oven for 48 hours before storage in a 50 ml polypropylene screw capped tube.

### EXAMPLE 3

### Recrystallization from Methylisobutyl Ketone ("MIBK")

An equal mixture (1:1 by weight) of FCP-3P1 and FCP-3P2 was dissolved in MIBK at ambient temperature to achieve saturation solution. Phytosterol was recrystallized from the saturated MIBK solution at approximately 6° C overnight before being filtered onto No.1 Whatman filter paper using a Buchner glass filter funnel with vacuum suction. The recrystallized material was dried at 35° C under open atmosphere in a hot oven for 48 hours before storage in a 50 ml polypropylene screw capped tube.

### EXAMPLE 4

### Recrystallization from Toluene

An equal mixture (1:1 by weight) of FCP-3P1 and FCP-3P2 was dissolved in toluene at ambient temperature to achieve saturation solution. Phytosterol was recrystallized from the saturated toluene solution at approximately 6° C overnight before being filtered onto No.1 Whatman filter paper using a Buchner glass filter funnel with vacuum suction. The recrystallized material was dried at 35° C under open atmosphere in a hot oven for 48 hours before storage in a 50 ml polypropylene screw capped tube.

### EXAMPLE 5

### Recrystallization from Hexane/Acetone

An equal mixture (1:1 by weight) of FCP-3P1 and FCP-3P2 was dissolved in a hexane/acetone blend (50% of each w/w) at ambient temperature to achieve saturation solution. Phytosterol was recrystallized from the saturated hexane/acetone solution at approximately 6° C overnight before being filtered onto No.1 Whatman filter paper using a Buchner glass filter funnel with vacuum suction. The recrystallized material was dried at 35° C under open atmosphere in a hot oven for 48 hours before storage in a 50 ml polypropylene screw capped tube.

### EXAMPLE 6

Purification and Hydrogenation of Crude Tall Oil Phytosterols, and Subsequent Blending or "Spiking" using Recrystallization Process
Goal: The therapeutic composition to be prepared had to fall within the following predetermined specifications:

| | |
|---|---|
| Beta-sitosterol | 38-60% |
| Sitostanol | 14-34% |
| Campesterol | 9-28% |
| Campestanol | 2-14% |

a) Purification: crude tall oil sterols (55 kg) were dispersed into 220 L of isopropyl alcohol ("IPA"). Activated carbon (5.5 kg) was added to purify the sterols. The purification was carried out by agitating the mixture for 2 hours at 65° C. The carbon was filtered out using a Mott filter, also at 65° C.
b) Hydrogenation:A portion (30% w/w) of the purified phytosterol (59.41% beta-sitosterol; 4.73% sitostanol; 24.19% campesterol; and 7.39% campestanol, all % w/w) mixture was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated portion (74.47% sitostanol and 20.35% campestanol) and the non-hydrogenated portion (70% w/w with specification noted above) were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w):

| | |
|---|---|
| Beta-sitosterol | 41.59% |
| Sitostanol | 25.65% |
| Campesterol | 16.93% |
| Campestanol | 11.28% |

The sitostanol concentration is significantly higher in the recrystallized product than the originally purified material (25.65% as compared to 4.73%) which translates into a product of higher therapeutic efficacy.

### EXAMPLE 7

### Purification and Hydrogenation of Crude Vegetable Derived Phytosterols, and Subsequent Blending or "Spiking" using Recrystallization Process

Goal: The therapeutic composition to be prepared had to fall within the following predetermined specifications:

| | |
|---|---|
| Beta-sitosterol | 18-60% |
| Sitostanol | 14-34% |
| Campesterol | 9-28% |
| Campestanol | 2-14% |
| Stigmasterol | n/a |
| Brassicasterol | n/a |

a) Purification: Soy sterols were purchased commercially from ADM.
b) Hydrogenation: A portion (44% w/w) of the purified phytosterols (45.70% beta-sitosterol; 2.1% sitostanol; 27.90% campesterol; and 14.80% stigmasterol and 5.3% brassicasterol, all % w/w) mixture was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated portion (with a specification of 62.60% sitostanol and 33.20% campestanol) and the non-hydrogenated portion (in an amount of 56% w/w and having the specification noted above) were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 25.60% |
| Sitostanol | 28.70% |
| Campesterol | 15.60% |
| Campestanol | 14.60% |
| Stigmasterol | 8.30% |
| Brassicasterol | 3.00% |

As in Example 6 above, the sitostanol concentration is significantly higher in the recrystallized product than the originally purified material (25.60% as compared to 2.10%) which translates into a product of higher therapeutic efficacy.

### EXAMPLE 8

### Purification and Hydrogenation of Crude Vegetable Derived Phytosterols, and Subsequent Blending or "Spiking" with Tall Oil Derived Phytosterols using Recrystallization Process

Goal: The therapeutic composition to be prepared had to fall within the following predetermined specifications:

| | |
|---|---|
| Beta-sitosterol | 18-60% |
| Sitostanol | 14-34% |
| Campesterol | 9-28% |
| Campestanol | 10-30% |
| Stigmasterol | n/a |
| Brassicasterol | n/a |

a) Purification: Vegetable sterols were purchased commercially from Henkel.
b) Hydrogenation: The vegetable phytosterol mixture was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated sterols (with a specification of 0% beta-sitosterol; 40% sitostanol; 0% campesterol; 40% campestanol and 0% brassicasterol, all % w/w) and the non-hydrogenated tall oil derived sterols (with a specification of 59.4% beta-sitosterol; 4.7% sitostanol; 24.2% campesterol; 7.4% campestanol and 0% brassicasterol, all % w/w) at a ratio of 55:45 % w/w were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 26.7% |
| Sitostanol | 24.1% |
| Campesterol | 10.9% |
| Campestanol | 25.3% |
| Brassicasterol | 0% |

### EXAMPLE 9

### Purification and Hydrogenation of Crude Tall Oil Derived Phytosterols, and Subsequent Blending or "Spiking" with Vegetable Derived Phytosterols using Recrystallization Process

Goal: The therapeutic composition to be prepared had to fall within the following predetermined specifications:

| | |
|---|---|
| Beta-sitosterol | 18-60% |
| Sitostanol | 14-34% |
| Campesterol | 9-28% |
| Campestanol | 2-30% |
| Stigmasterol | n/a |
| Brassicasterol | n/a |

a) Purification: crude tall oil sterols (55 kg) were dispersed into 220 L of isopropyl alcohol ("IPA"). Activated carbon (5.5 kg) was added to purify the sterols. The purification was carried out by agitating the mixture for 2 hours at 65° C. The carbon was filtered out using a Mott filter, also at 65° C.
b) Hydrogenation: The tall oil phytosterols were hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated sterols (with a specification of 0% beta-sitosterol; 74.5% sitostanol; 0% campesterol; 20.4% campestanol and 0% brassicasterol, all % w/w) and the non-hydrogenated vegetable derived derived sterols, purchased commercially from Henkel (with a specification of 40% beta-sitosterol; 0% sitostanol; 33% campesterol; 0% campestanol and 7% brassicasterol, all % w/w) at a ratio of 33:67 % w/w were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 26.8% |
| Sitostanol | 24.6% |
| Campesterol | 22.1% |
| Campestanol | 6.7% |
| Brassicasterol | 4.7% |

### EXAMPLE 10

### Purification and Hydrogenation of Crude Vegetable Derived Phytosterols, and Subsequent Blending or "Spiking" with Vegetable Derived Phytosterols using Recrystallization Process

Goal: The therapeutic composition to be prepared had to fall within the following predetermined specifications:

| | |
|---|---|
| Beta-sitosterol | 18-60% |
| Sitostanol | 14-34% |
| Campesterol | 9-28% |
| Campestanol | 10-30% |
| Stigmasterol | n/a |
| Brassicasterol | n/a |

a) Purification: Vegetable sterols were purchased commercially from Henkel and ADM.
b) Hydrogenation: The vegetable phytosterol mixture from ADM was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated sterols (with a specification of 0% beta-sitosterol; 55.6% sitostanol; 0% campesterol; 23.9% campestanol, 6.9% brassicastanol and 0% brassicasterol, all % w/w) and the non-hydrogenated vegetable derived sterols from Henkel (with a specification of 40% beta-sitosterol; 0% sitostanol; 33% campesterol; 0% campestanol and 7% brassicasterol, all % w/w) at a ratio of 44:56 % w/w were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 22.4% |
| Sitostanol | 24.5% |
| Campesterol | 18.5% |
| Campestanol | 10.5% |
| Brassicasterol | 3.9% |
| Brassicastanol | 3.0% |

### EXAMPLE 11

### Purification and Hydrogenation of Crude Vegetable Derived Phytosterols, and Subsequent Blending or "Spiking" with Vegetable Derived Phytosterols using Recrystallization Process

Goal: The therapeutic composition to be prepared had to fall within the following predetermined specifications:

| | |
|---|---|
| Beta-sitosterol | 10-60% |
| Sitostanol | 14-34% |
| Campesterol | 5-28% |
| Campestanol | 10-30% |
| Stigmasterol | n/a |
| Brassicasterol | n/a |

a) Purification: Vegetable sterols were purchased commercially from Henkel.
b) Hydrogenation: The vegetable phytosterol mixture was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated sterols (with a specification of 0% beta-sitosterol; 40% sitostanol; 0% campesterol; 39% campestanol, and 0% brassicasterol, all % w/w) and the non-hydrogenated vegetable derived sterols (with a specification of 40% beta-sitosterol; 0% sitostanol; 33% campesterol; 0% campestanol and 6% brassicasterol, all % w/w) at a ratio of 73:27 % w/w were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 10.8% |
| Sitostanol | 29.2% |
| Campesterol | 8.9% |
| Campestanol | 28.5% |
| Brassicasterol | 1.6% |

### EXAMPLE 12

### Purification and Hydrogenation of Crude Vegetable Derived Phytosterols, and Subsequent Blending or "Spiking" with Vegetable Derived Phytosterols using Recrystallization Process

Goal: The therapeutic composition to be prepared had to fall within the following predetermined specifications:

| | |
|---|---|
| Beta-sitosterol | 10-60% |
| Sitostanol | 14-34% |
| Campesterol | 5-28% |
| Campestanol | 10-30% |
| Stigmasterol | n/a |
| Brassicasterol | n/a |

a) Purification: Vegetable sterols were purchased commercially from Cargill.
b) Hydrogenation: The vegetable phytosterol mixture was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated sterols (with a specification of 0% beta-sitosterol; 67% sitostanol; 0% campesterol; 25.6% campestanol, and 0% brassicasterol, all % w/w) and the non-hydrogenated vegetable derived sterols (with a specification of 43% beta-sitosterol; 1.0% sitostanol; 24% campesterol; 0.8% campestanol, 23% stigmasterol and 0.8% brassicasterol, all % w/w) at a ratio of 49:51 % w/w were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 21.9% |
| Sitostanol | 33.3% |
| Stigmasterol | 11.7% |
| Campesterol | 12.2% |
| Campestanol | 1 3% |
| Brassicasterol | 0.4% |

### EXAMPLE 13

### Purification and Hydrogenation of Crude Vegetable Derived Phytosterols, and Subsequent Blending or "Spiking" with Vegetable Derived Phytosterols using Recrystallization Process

Goal: The therapeutic composition to be prepared had to fall within the following predetermined specifications:

| | |
|---|---|
| Beta-sitosterol | 10-60% |
| Sitostanol | 14-34% |
| Campesterol | 5-28% |
| Campestanol | 10-30% |
| Stigmasterol | n/a |
| Brassicasterol | n/a |

a) Purification: Vegetable sterols were purchased commercially from Cargill and Henkel.
b) Hydrogenation: The vegetable phytosterol mixture from Henkel was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated sterols (with a specification of 0% beta-sitosterol; 40% sitostanol; 0% campesterol; 36-44% campestanol, and 0% brassicasterol, all % w/w) and the non-hydrogenated vegetable derived sterols from Cargill (with a specification of 43% beta-sitosterol; 1.0% sitostanol; 24% campesterol; 0.8% campestanol, 23% stigmasterol and 0.8% brassicasterol, all % w/w) at a ratio of 72:28 % w/w were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 12% |
| Sitostanol | 29.1 % |
| Stigmasterol | 6.4% |
| Campesterol | 6.7% |
| Campestanol | 26.1-31.9% |
| Brassicasterol | 0.2% |

### EXAMPLE 14

### Purification and Hydrogenation of Crude Vegetable Derived Phytosterols, and Subsequent Blending or "Spiking" with Vegetable Derived Phytosterols using Recrystallization Process

Goal: The therapeutic composition to be prepared had to fall within the following predetermined specifications:

| | |
|---|---|
| Beta-sitosterol | 5-60% |
| Sitostanol | 14-34% |
| Campesterol | 5-28% |
| Campestanol | 10-40% |
| Stigmasterol | n/a |
| Brassicasterol | 1-5% |

a) Purification: Vegetable sterols were purchased commercially from Henkel.
b) Hydrogenation: The vegetable phytosterol mixture was hydrogenated in IPA using palladium on alumina catalyst. The hydrogenation process was carried out at 70° C and 60psi hydrogen pressure for 3 hours. A Mott filter was used to recover the palladium catalyst after hydrogenation.
c) Blending/Spiking: The hydrogenated sterols (with a specification of 0% beta-sitosterol; 40% sitostanol; 0% campesterol; 36-44% campestanol, and 0% brassicasterol, all % w/w) and the non-hydrogenated vegetable derived sterols (with a specification of 40% beta-sitosterol; 0% sitostanol; 30-36% campesterol; 0% campestanol and 6-8% brassicasterol, all % w/w) at a ratio of 83:17 % w/w were dissolved in IPA solution at 75° C to achieve saturation solution. The product was recrystallized from the saturated isopropyl alcohol solution at approximately 6° C overnight before being filtered onto No.1 Whatman filter paper using a Buchner glass filter funnel with vacuum suction. The recrystallized material was then dried at 35° C under open atmosphere in a hot oven for 48 hours before storage in a 50 ml polypropylene screw capped tube. The recrystallized product so formed had the following specifications (% w/w) which fell satisfactorily within the desired ranges:

| | |
|---|---|
| Beta-sitosterol | 6.8% |
| Sitostanol | 33.2% |
| Stigmasterol | 0% |
| Campesterol | 5.1-6.1 % |
| Campestanol | 29.9-36.5% |
| Brassicasterol | 1-4% |

### REFERENCES

1. Law M.R., Wald N.J., Wu., Hacksaw ZA., Bailey A.; Systemic underestimation of association between serum cholesterol concentration and ischemic heart disease in observational studies: Data from BUPA Study; *Br. Med. J*. 1994; 308:363-366
2. Law M.R., Wald N.J., Thompson S.G.; By how much and how quickly does reduction in serum cholesterol concentration lower risk of ischemic heart disease? *Br. Med. J.* 1994; 308:367-373
3. La Rosa J.C., Hunninghake D.. Bush D. et al.; The cholesterol facts: A summary of the evidence relating to dietary fats, serum cholesterol and coronary heart disease:Ajoint statement by the American Heart Association and the National Heart, Lung and Blood Institute. *Circulation* 1990; 81:1721-1733
4. Havel R.J., Rapaport E.. Drug Therapy: Management of Primary Hyperlipidemia. *New England Journal of Medicine,* 1995; 332:1491-1498
5. Kuccodkar et al.; Effects of plant sterols on cholesterol metabolism. *Atherosclerosis,* 1976; 23:239-248
6. Lees R.S., Lees A.M. Effects of sitosterol therapy on plasma lipid and lipoprotein concentrations. In: Greten H (Ed) Lipoprotein Metabolism. Springer-Verlag, Berlin, Heidelberg, New York, 1976:119-124
7. Lees A.M., Mok H.Y.I., Lees R.S., McCluskey M.A., Grundy S.M. Plant sterols as cholesterol-lowering agents: clinical trials in patients with hypercholesterolemia and studies of sterol balance. *Atherosclerosis* 1977; 28: 325-338
8. Mattson FH et al.: *American Journal of Clinical Nutrition.* 35(4): 697-700, 1982
9. Gyling H.K. et al. *Circulation* 1996; 6:1-578
10 Hassan A.S. and Rampone A.J. *J.-Lipid Res.* 1979:20; 646-653
11.Lees R.S, Lees A.M. Effects of sitosterol therapy on plasma lipids and lipoprotein concentrations, pp 119-124 in "Lipoprotein Metabolism" Ed Greten H., Berlin, Heidelburg, NY: Springer-Verlag, 1976
12. Mattson F.H., Volpenhein R.A. and Erickson B.A. *J*. *Nutrition* 1977:107; 1109-1146
13.Heinemann et al. *Agents Actions(Suppl)* 1988:26; 117-122

## Claims

1. A composite crystalline structure comprising a phytosterol or a derivative thereof and a phytostanol or a derivative thereof prepared by:
a) dissolving a phytosterol or esterified derivative thereof purified from its source and a phytostanol or esterified derivative thereof purified from its source in a solvent at an ambient temperature or temperature above ambient but lower than the boiling point of the solvent;
b) cooling the solvent to allow crystal formation; and
c) filtering and washing the crystals so formed; wherein said structure has a single composite endotherm as evidenced using differential scanning calorimetry.

2. The structure of claim 1 wherein the phytosterol is selected from the group consisting of sitosterol, campesterol, stigmasterol, brassicasterol, desmosterol, chalinosterol, poriferasterol, clionasterol and all natural or synthesized forms thereof, including isomers.

3. The structure of claim 1 wherein the phytostanol is selected from the group consisting of all saturated or hydrogenated phytosterols and all natural or synthesized forms thereof, including isomers.

4. The structure of any preceding claim for use in a method of treatment of an animal.

5. Use of a structure according to any preceding claim in the manufacture of a product for use in a method of treating or preventing cardiovascular disease and its underlying conditions including atherosclerosis and hypercholesterolemia in an animal.

6. Use of claim 5 wherein the animal is human.

7. Use according to claim 5 or claim 6 wherein in the method the structure lowers serum cholesterol.

8. A product which comprises a composite crystalline structure according to any of claims 1 to 3 and a pharmaceutically acceptable or food-grade carrier therefor, for administration to an animal.

9. A product according to claim 8 which is a comestible or beverage.

10. A process of making a composite crystalline structure comprising a phytosterol and a phytostanol wherein said structure has a single composite endotherm as evidenced using differential scanning calorimetry, which comprises :
a) dissolving the phytosterol or esterified derivative thereof purified from its source and the phytostanol or esterified derivative thereof purified from its source in a solvent at an ambient temperature or temperature above ambient but lower than the boiling point of the solvent;
b) cooling the solvent to allow crystal formation; and
c) filtering and washing the crystals so formed.

11. The process of claim 10 wherein the solvent is any hydrocarbon having from 5 to 10 carbon atoms.

12. The process of claim 10 wherein the solvent is any alcohol having from 1 to 10 carbon atoms.

13. The process of claim 10 wherein the solvent is aromatic.

14. The process of claim 10 wherein the solvent is any ketone having from 1 to 10 carbon atoms.

15. The process of claim 10 wherein the solvent is any ester derived from carboxylic acids having from 2 to 10 carbon atoms, preferably ethyl acetate.

16. The process of claim 10 wherein the phytosterol or esterified derivative thereof rest and phytostanol or esterified derivative thereof are dissolved in the solvent at a temperature of between 18 and 80°C.

17. The process of claim 10 wherein the solvent is cooled, at step b) to a temperature of between 1 and 20°C.

18. The process of claim 10 wherein the phytosterol is selected from the group consisting of sitosterol, campesterol, stigmasterol, brassicasterol, desmosterol, chalinosterol, poriferasterol, clionasterol and all natural or synthesized forms and esterified derivatives thereof, including isomers.

19. The process of claim 10 wherein the phytostanol is selected from the group consisting of all saturated or hydrogenated phytosterols and all natural or synthesized forms and esterified derivatives thereof, including isomers.

## Patentansprüche

1. Kristalline Kompositstruktur, umfassend ein Phytosterol oder ein Derivat davon und ein Phytostanol oder ein Derivat davon, hergestellt durch:
a) Lösen eines Phytosterols oder eines veresterten Derivats davon, gereinigt von seiner Quelle, und eines Phytostanols oder eines veresterten Derivats davon, gereinigt von seiner Quelle, in einem Lösungsmittel bei Umgebungstemperatur oder einer Temperatur oberhalb der Umgebungstemperatur, aber niedriger als dem Siedepunkt des Lösungsmittels;
b) Kühlen des Lösungsmittels, um Kristallbildung zu ermöglichen; und
c) Filtern und Waschen der so gebildeten Kristalle; wobei die Struktur eine einzelne Kompositendotherme aufweist, wie durch Differentialscanningkalorimetrie belegt.

2. Struktur nach Anspruch 1, bei der das Phytosterol ausgewählt ist aus der Gruppe bestehend aus Sitosterol, Campesterol, Stigmasterol, Brassicasterol, Desmosterol, Chalinosterol, Poriferasterol, Clionasterol und allen natürlichen oder synthetisierten Formen davon, einschließlich Isomeren.

3. Struktur nach Anspruch 1, bei der das Phytostanol ausgewählt ist aus der Gruppe bestehend aus allen gesättigten oder hydrierten Phytosterolen und allen natürlichen oder synthetisierten Formen davon, einschließlich Isomeren.

4. Struktur nach irgendeinem vorhergehenden Anspruch zur Verwendung in einem Verfahren zur Behandlung eines Tieres.

5. Verwendung einer Struktur nach irgendeinem vorhergehenden Anspruch bei der Herstellung eines Produkts zur Verwendung in einem Verfahren zur Behandlung oder Vermeidung von kardiovaskulärer Erkrankung oder deren zugrunde liegenden Zuständen, einschließlich Arteriosklerose und Hypercholesterinämie bei einem Tier.

6. Verwendung nach Anspruch 5, wobei das Tier ein Mensch ist.

7. Verwendung nach Anspruch 5 oder Anspruch 6, wobei die Struktur in dem Verfahren das Serumcholesterin senkt.

8. Produkt, welches umfasst eine kristalline Kompositstruktur nach irgendeinem der Ansprüche 1 bis 3 und einen pharmazeutisch verträglichen oder nahrungsmittelgeeigneten Träger dafür, zur Verabreichung an ein Tier.

9. Produkt nach Anspruch 8, bei dem es sich um ein Nahrungsmittel oder ein Getränk handelt.

10. Verfahren zur Herstellung einer kristallinen Kompositstruktur, umfassend ein Phytosterol und ein Phytostanol, wobei die Struktur eine einzelne Kompositendotherme aufweist, wie durch Differentialscanningkalorimetrie belegt, welches umfasst:
a) Lösen des Phytosterols oder eines veresterten Derivats davon, gereinigt von seiner Quelle, und des Phytostanols oder eines veresterten Derivats davon, gereinigt von seiner Quelle, in einem Lösungsmittel bei Umgebungstemperatur oder einer Temperatur oberhalb der Umgebungstemperatur, aber niedriger als dem Siedepunkt des Lösungsmittels;
b) Kühlen des Lösungsmittels, um Kristallbildung zu ermöglichen; und
c) Filtern und Waschen der so gebildeten Kristalle.

11. Verfahren nach Anspruch 10, bei dem das Lösungsmittel ein Kohlenwasserstoff mit 5 bis 10 Kohlenstoffatomen ist.

12. Verfahren nach Anspruch 10, bei dem das Lösungsmittel ein Alkohol mit 1 bis 10 Kohlenstoffatomen ist.

13. Verfahren nach Anspruch 10, bei dem das Lösungsmittel aromatisch ist.

14. Verfahren nach Anspruch 10, bei dem das Lösungsmittel ein Keton mit 1 bis 10 Kohlenstoffatomen ist.

15. Verfahren nach Anspruch 10, bei dem das Lösungsmittel ein Ester ist, der von Carbonsäuren mit 2 bis 10 Kohlenstoffatomen abgeleitet ist, bevorzugt Ethylacetat.

16. Verfahren nach Anspruch 10, bei dem das Phytosterol oder das veresterte Derivat davon und das Phytostanol oder das veresterte Derivat davon in dem Lösungsmittel bei einer Temperatur zwischen 18 und 80°C gelöst werden.

17. Verfahren nach Anspruch 10, bei dem das Lösungsmittel in Schritt b) auf eine Temperatur zwischen 1 und 20°C gekühlt wird.

18. Verfahren nach Anspruch 10, bei dem das Phytosterol ausgewählt ist aus der Gruppe bestehend aus Sitosterol, Campesterol, Stigmasterol, Brassicasterol, Desmosterol, Chalinosterol, Poriferasterol, Clionasterol und allen natürlichen oder synthetisierten Formen und veresterten Derivaten davon, einschließlich Isomeren.

19. Verfahren nach Anspruch 10, bei dem das Phytostanol ausgewählt ist aus der Gruppe bestehend aus allen gesättigten oder hydrierten Phytosterolen und allen natürlichen oder synthetisierten Formen und veresterten Derivaten davon, einschließlich Isomeren.

## Revendications

1. Structure cristalline composite, comprenant un phytostérol ou un dérivé de celui-ci et un phytostanol ou un dérivé de celui-ci, préparée par :
a) dissolution d'un phytostérol ou d'un dérivé estérifié de celui-ci, purifié de sa source et d'un phytostanol ou d'un dérivé estérifié de celui-ci, purifié de sa source, dans un solvant à température ambiante ou à une température supérieure à la température ambiante, mais inférieure au point d'ébullition du solvant ;
b) refroidissement du solvant pour permettre la formation de cristaux, et
c) filtration et lavage des cristaux ainsi formés, où ladite structure a une courbe endothermique composite unique mise en évidence par calorimétrie différentielle à balayage.

2. Structure selon la revendication 1, dans laquelle le phytostérol est choisi parmi le groupe consistant en le sitostérol, le campestérol, le stigmastérol, le brassicastérol, le desmostréol, le chalinostérol, le poriférastérol, le clionastérol et toutes les formes naturelles ou synthétisées de ceux-ci, y compris les isomères.

3. Structure selon la revendication 1, dans laquelle le phytostanol est choisi parmi le groupe consistant en tous les phytostérols saturés ou hydrogénés et toutes les formes naturelles ou synthétisées de ceux-ci, y compris les isomères.

4. Structure selon l'une quelconque des revendications précédentes, à utiliser dans un procédé de traitement d'un animal.

5. Utilisation d'une structure selon l'une quelconque des revendications précédentes, dans la préparation d'un produit à utiliser dans un procédé de traitement ou de prévention d'une maladie cardiovasculaire et de ses états sous-jacents, y compris l'athérosclérose et l'hypercholestérolémie chez un animal.

6. Utilisation selon la revendication 5, dans laquelle l'animal est un humain.

7. Utilisation selon la revendication 5 ou 6, dans laquelle, dans le procédé, la structure diminue le cholestérol sérique.

8. Produit, qui comprend une structure cristalline composite selon l'une quelconque des revendications 1 à 3 et un support pharmaceutique acceptable ou de qualité alimentaire, à administrer à un animal.

9. Produit selon la revendication 8, qui est un aliment ou une boisson.

10. Procédé de préparation d'une structure cristalline composite, comprenant un phytostérol et un phytostanol, dans lequel ladite structure a une courbe endothermique composite unique mise en évidence par calorimétrie différentielle à balayage, qui comprend :
a) la dissolution du phytostérol ou d'un dérivé estérifié de celui-ci, purifié de sa source et du phytostanol ou d'un dérivé estérifié de celui-ci, purifié de sa source, dans un solvant à température ambiante ou à une température supérieure à la température ambiante, mais inférieure au point d'ébullition du solvant ;
b) le refroidissement du solvant pour permettre la formation de cristaux, et
c) la filtration et le lavage des cristaux ainsi formés.

11. Procédé selon la revendication 10, dans lequel le solvant est un hydrocarbure quelconque, ayant de 5 à 10 atomes de carbone.

12. Procédé selon la revendication 10, dans lequel le solvant est un alcool quelconque, ayant de 1 à 10 atomes de carbone.

13. Procédé selon la revendication 10, dans lequel le solvant est aromatique.

14. Procédé selon la revendication 10, dans lequel le solvant est une cétone quelconque, ayant de 1 à 10 atomes de carbone.

15. Procédé selon la revendication 10, dans lequel le solvant est un ester quelconque, dérivé d'acides carboxyliques ayant de 2 à 10 atomes de carbone, de préférence l'acétate d'éthyle.

16. Procédé selon la revendication 10, dans lequel le phytostérol ou son dérivé estérifié et le phytostanol ou son dérivé estérifié sont dissous dans le solvant à une température allant de 18 à 80°C.

17. Procédé selon la revendication 10, dans lequel le solvant est refroidi, à l'étape b), jusqu'à une température allant de 1 à 20°C.

18. Procédé selon la revendication 10, dans lequel le phytostérol est choisi parmi le groupe consistant en le sitostérol, le campestérol, le stigmastérol, le brassicastérol, le desmostréol, le chalinostérol, le poriférastérol, le clionastérol et toutes les formes naturelles ou synthétisées et dérivés estérifiés de ceux-ci, y compris les isomères.

19. Procédé selon la revendication 10, dans lequel le phytostanol est choisi parmi le groupe consistant en tous les phytostérols saturés ou hydrogénés et toutes les formes naturelles ou synthétisées et dérivés estérifiés de ceux-ci, y compris les isomères.
